# EUROPEAN PATENT SPECIFICATION

(11) **EP 2 236 514 B1**
(45) Date of publication and mention of the grant of the patent: **03.04.2013**
(21) Application number: 09704366.5
(22) Date of filing: 23.01.2009
(51) Int. Cl.: C07K 7/08, C12N 5/00

(54) **ARTIFICIAL PEPTIDE AND USE THEREOF**
KÜNSTLICHES PEPTID UND VERWENDUNG DAVON
PEPTIDE ARTIFICIEL ET SON UTILISATION

(30) Priority: 25.01.2008 JP 2008014966
(43) Date of publication of application: 06.10.2010
(73) Proprietor: Toagosei Co., Ltd, Minato-ku, Tokyo 105-8419 (JP)
(72) Inventor: YOSHIDA, Tetsuhiko, Tsukuba-shi Ibaraki 300-2611 (JP); KOBAYASHI, Nahoko, Tsukuba-shi Ibaraki 300-2611 (JP); MASUDA, Junichi, Tsukuba-shi Ibaraki 300-2611 (JP)
(74) Representative: Elsy, David
(86) International application number: PCT/JP2009/051082
(87) International publication number: WO 2009/093692

(56) References cited:
- WO-A1-2004/056854
- WO-A1-2007/010989
- WO-A1-2007/010989
- JP-A- 2007 145 761
- GOYAL PANKAJ ET AL: "Phosphorylation-dependent regulation of unique nuclear and nucleolar localization signals of LIM kinase 2 in endothelial cells.", THE JOURNAL OF BIOLOGICAL CHEMISTRY 1 SEP 2006 LNKD- PUBMED:16820362, vol. 281, no. 35, 1 September 2006 (2006-09-01), pages 25223-25230, XP002622536, ISSN: 0021-9258
- TAKEI Y. ET AL.: "Possible involvement of a pertussis toxin-sensitive GTP-binding protein in protein transport into nuclei isolated from rat liver", J. BIOCHEM., vol. 115, no. 3, 1994, pages 578-583, XP002622537,
- CSERPAN I. ET AL.: "The mechanism of nuclear transport of natural or artificial transport substrates in digitonin-permeabilized cells", J. CELL. SCI., vol. 108, no. 5, 1995, pages 1849-1861, XP002622538,
- KOBAYASHI NAHOKO ET AL: "Nucleolar Localization Signals of LIM Kinase 2 Function as a Cell-Penetrating Peptide", PROTEIN AND PEPTIDE LETTERS, BENTHAM SCIENCE PUBLISHERS, NL, vol. 17, no. 12, 1 December 2010 (2010-12-01), pages 1480-1488, XP009144516, ISSN: 0929-8665
- GOYAL P. ET AL.: 'Phosphorylation-dependent regulation of unique nuclear and nucleolar localization signals of LIM kinase 2 in endothelial cells' J. BIOL. CHEM. vol. 281, no. 35, 2006, pages 25223 - 25230
- TAKEI Y. ET AL.: 'Possible involvement of a pertussis toxin-sensitive GTP-binding protein in protein transport into nuclei isolated from rat liver' J. BIOCHEM. vol. 115, no. 3, 1994, pages 578 - 583
- CSERPAN I. ET AL.: 'The mechanism of nuclear transport of natural or artificial transport substrates in digitonin-permeabilized cells' J. CELL. SCI. vol. 108, no. 5, 1995, pages 1849 - 1861
- SHIRO FUTAKI: 'Maku Toka Peptide o Mochiita Tanpakushitsu Saibo Nai Iso' KAGAKU TO SEIBUTSU vol. 43, no. 10, 2005, pages 649 - 653, XP003011960

### SEQUENCE LISTING

<110> TOAGOSEI CO., LTD
<120> ARTIFICIAL PEPTIDES AND USE THEREOF
<130> TG08-001PCT
<140> JP2008-014966
   <141> 2008-01-25
<160> 85
<170> PatentIn version 3.1
<210> 1
   <211> 13
   <212> PRT
   <213> Artificial sequence
<220>
   <223> Synthetic peptide
<400> 1
   Lys Lys Arg Thr Leu Arg Lys Asn Asp Arg Lys Lys Arg
   1 5 10
<210> 2
   <211> 15
   <212> PRT
   <213> Artificial sequence
<220>
   <223> Synthetic peptide
<400> 2
   Pro Leu Gln Glu Leu Cys Arg Gln Arg Ile Val Ala Ala Val Gly
   1 5 10 15
<210> 3
   <211> 15
   <212> PRT
   <213> Artificial sequence
<220>
   <223> Synthetic peptide
<400> 3
   Thr Leu Gln His Phe Cys Arg Leu Ala Ile Asn Lys Cys Thr Gly
   1 5 10 15
<210> 4
   <211> 15
   <212> PRT
   <213> Artificial sequence
<220>
   <223> Synthetic peptide
<400> 4
   Thr Leu Gln His Leu Cys Arg Lys Thr Val Asn Gly His Leu Asp
   1 5 10 15
<210> 5
   <211> 15
   <212> PRT
   <213> Artificial sequence
<220>
   <223> Synthetic peptide
<400> 5
   Ser Leu Gln His Ile Cys Arg Thr Val Ile Cys Asn Cys Thr Thr
   1 5 10 15
<210> 6
   <211> 15
   <212> PRT
   <213> Artificial sequence
<220>
   <223> Synthetic peptide
<400> 6
   Ser Leu Gln Tyr Ile Cys Arg Ala Val Ile Cys Arg Cys Thr Thr
   1 5 10 15
<210> 7
   <211> 15
   <212> PRT
   <213> Artificial sequence
<220>
   <223> Synthetic peptide
<400> 7
   Ser Leu Gln Tyr Leu Cys Arg Phe Val Ile Arg Gln Tyr Thr Arg
   1 5 10 15
<210> 8
   <211> 15
   <212> PRT
   <213> Artificial sequence
<220>
   <223> Synthetic peptide
<400> 8
   Ser Leu Gln His Leu Cys Arg Phe Arg Ile Arg Gln Leu Val Arg
   1 5 10 15
<210> 9
   <211> 15
   <212> PRT
   <213> Artificial sequence
<220>
   <223> Synthetic peptide
<400> 9
   Ser Leu Gln Asp Leu Cys Cys Arg Ala Val Val Ser Cys Thr Pro
   1 5 10 15
<210> 10
   <211> 15
   <212> PRT
   <213> Artificial sequence
<220>
   <223> Synthetic peptide
<400> 10
   Ser Leu Gln Asp Leu Cys Cys Arg Thr Ile Val Ser Cys Thr Pro
   1               5                   10                  15
<210> 11
   <211> 15
   <212> PRT
   <213> Artificial sequence
<220>
   <223> Synthetic peptide
<400> 11
   Ser Leu Gln His Ile Cys Arg Met Ser Ile Arg Arg Val Met Ser
   1               5                   10                  15
<210> 12
   <211> 15
   <212> PRT
   <213> Artificial sequence
<220>
   <223> Synthetic peptide
<400> 12
   Ser Leu Lys His Leu Cys Arg Lys Ala Leu Arg Ser Phe Leu Thr
   1               5                   10                  15
<210> 13
   <211> 15
   <212> PRT
   <213> Artificial sequence
<220>
   <223> Synthetic peptide
<400> 13
   Thr Leu Leu Ser Leu Cys Arg Val Ala Val Arg Arg Ala Leu Gly
   1               5                   10                  15
<210> 14
   <211> 15
   <212> PRT
   <213> Artificial sequence
<220>
   <223> Synthetic peptide
<400> 14
   Pro Leu Ala His Leu Cys Arg Leu Arg Val Arg Lys Ala Ile Gly
   1 5 10 15
<210> 15
   <211> 15
   <212> PRT
   <213> Artificial sequence
<220>
   <223> Synthetic peptide
<400> 15
   Ser Leu Thr His Leu Cys Arg Leu Glu Ile Arg Ser Ser Ile Lys
   1 5 10 15
<210> 16
   <211> 15
   <212> PRT
   <213> Artificial sequence
<220>
   <223> Synthetic peptide
<400> 16
   Thr Leu Leu Glu Ser Ser Ala Arg Thr Ile Leu His Asn Arg Ile
   1 5 10 15
<210> 17
   <211> 15
   <212> PRT
   <213> Artificial sequence
<220>
   <223> Synthetic peptide
<400> 17
   Asn Leu Gln Asp Leu Cys Arg Ile Lys Ile Arg Gln Cys Ile Gly
   1 5 10 15
<210> 18
   <211> 15
   <212> PRT
   <213> Artificial sequence
<220>
   <223> Synthetic peptide
<400> 18
   Ser Leu Gln His Leu Cys Arg Cys Ala Leu Arg Ser His Leu Glu
   1 5 10 15
<210> 19
   <211> 15
   <212> PRT
   <213> Artificial sequence
<220>
   <223> Synthetic peptide
<400> 19
   Ser Leu Lys His Leu Cys Arg Leu Lys Ile Arg Lys Cys Met Gly
   1               5                   10                  15
<210> 20
   <211> 15
   <212> PRT
   <213> Artificial sequence
<220>
   <223> Synthetic peptide
<400> 20
   Pro Leu Gln Glu Leu Cys Arg Gln Arg Ile Val Ala Thr Val Gly
   1               5                   10                  15
<210> 21
   <211> 15
   <212> PRT
   <213> Artificial sequence
<220>
   <223> Synthetic peptide
<400> 21
   Pro Leu Ala His Leu Cys Arg Leu Arg Val Arg Lys Ala Ile Gly
   1               5                   10                  15
<210> 22
   <211> 15
   <212> PRT
   <213> Artificial sequence
<220>
   <223> Synthetic peptide
<400> 22
   Ser Leu Gln His Leu Cys Arg Met Ser Ile Arg Arg Val Met Pro
   1               5                   10                  15
<210> 23
   <211> 15
   <212> PRT
   <213> Artificial sequence
<220>
   <223> Synthetic peptide
<400> 23
   Ser Leu Gln Asp Leu Cys Cys Arg Ala Val Val Ser Cys Thr Pro
   1               5                   10                  15
<210> 24
   <211> 15
   <212> PRT
   <213> Artificial sequence
<220>
   <223> Synthetic peptide
<400> 24
   Ser Leu Gln Tyr Leu Ala Leu Thr Ala Leu Ile Thr Pro Lys Lys
   1               5                   10                  15
<210> 25
   <211> 15
   <212> PRT
   <213> Artificial sequence
<220>
   <223> Synthetic peptide
<400> 25
   Ser Leu Gln Phe Leu Ala Leu Thr Val Tyr Thr Asp Phe Leu Arg
   1               5                   10                  15
<210> 26
   <211> 15
   <212> PRT
   <213> Artificial sequence
<220>
   <223> Synthetic peptide
<400> 26
   Ser Leu Gln Tyr Leu Ala Leu Arg Val Tyr Thr Asn Gly Leu Arg
   1               5                   10                  15
<210> 27
   <211> 15
   <212> PRT
   <213> Artificial sequence
<220>
   <223> Synthetic peptide
<400> 27
   Ser Leu Gln Leu Leu Ala Leu Val Ala Tyr Thr Asn Gly Ile Arg
   1               5                   10                  15
<210> 28
   <211> 15
   <212> PRT
   <213> Artificial sequence
<220>
   <223> Synthetic peptide
<400> 28
   Ser Leu Gln Tyr Leu Ala Leu Leu Ala His Gln Asn Gly Leu Arg
   1               5                   10                  15
<210> 29
   <211> 15
   <212> PRT
   <213> Artificial sequence
<220>
   <223> Synthetic peptide
<400> 29
   Ser Leu Gln Tyr Leu Ala Leu Gln Val Tyr Leu Lys Asp Gly Gly
   1               5                   10                  15
<210> 30
   <211> 15
   <212> PRT
   <213> Artificial sequence
<220>
   <223> Synthetic peptide
<400> 30
   Ser Leu Gln Tyr Leu Ala Ile Lys Ala Trp Ala Arg Gln Gln Leu
   1               5                   10                  15
<210> 31
   <211> 15
   <212> PRT
   <213> Artificial sequence
<220>
   <223> Synthetic peptide
<400> 31
   Ser Leu Gln Tyr Leu Ala Leu Lys Val Val Ser Asp Val Arg Ser
   1               5                   10                  15
<210> 32
   <211> 15
   <212> PRT
   <213> Artificial sequence
<220>
   <223> Synthetic peptide
<400> 32
   Ser Leu Gln Tyr Leu Ala Leu Thr Val Val Ser His Val Arg Ser
   1 5 10 15
<210> 33
   <211> 15
   <212> PRT
   <213> Artificial sequence
<220>
   <223> Synthetic peptide
<400> 33
   Ser Leu Gln Phe Leu Ala Leu Val Val Val Gln Gln Asn Gly Arg
   1 5 10 15
<210> 34
   <211> 15
   <212> PRT
   <213> Artificial sequence
<220>
   <223> Synthetic peptide
<400> 34
   Ser Leu Gln Phe Leu Ala Leu Arg Val Val Gln Glu Gly Lys Asn
   1 5 10 15
<210> 35
   <211> 15
   <212> PRT
   <213> Artificial sequence
<220>
   <223> Synthetic peptide
<400> 35
   Ser Leu Gln Phe Leu Ala Leu Gln Val Val Gln Lys Gly His Gly
   1 5 10 15
<210> 36
   <211> 15
   <212> PRT
   <213> Artificial sequence
<220>
   <223> Synthetic peptide
<400> 36
   Ser Leu Gln Phe Leu Cys Leu Arg Val Leu His Gly Gln Gln Glu
   1 5 10 15
<210> 37
   <211> 15
   <212> PRT
   <213> Artificial sequence
<220>
   <223> Synthetic peptide
<400> 37
   Ser Leu Gln Phe Leu Cys Leu Arg Gln Leu Gln His Val Gln Asn
   1 5 10 15
<210> 38
   <211> 15
   <212> PRT
   <213> Artificial sequence
<220>
   <223> Synthetic peptide
<400> 38
   Ser Leu Gln Phe Leu Cys Leu Arg Gln Leu Gln His Val Gln Ser
   1 5 10 15
<210> 39
   <211> 15
   <212> PRT
   <213> Artificial sequence
<220>
   <223> Synthetic peptide
<400> 39
   Ser Leu Gln Tyr Leu Cys Leu Arg Gln Leu Gln His Val Gln Thr
   1 5 10 15
<210> 40
   <211> 15
   <212> PRT
   <213> Artificial sequence
<220>
   <223> Synthetic peptide
<400> 40
   Ser Leu Gln Phe Ile Cys Leu Arg Gln Leu Gln His Val Gln Ala
   1 5 10 15
<210> 41
   <211> 15
   <212> PRT
   <213> Artificial sequence
<220>
   <223> Synthetic peptide
<400> 41
   Ser Leu Gln Phe Leu Cys Leu Arg Val Ile Tyr Gly Pro Glu Glu
   1 5 10 15
<210> 42
   <211> 15
   <212> PRT
   <213> Artificial sequence
<220>
   <223> Synthetic peptide
<400> 42
   Thr Leu Gln Phe Leu Cys Leu Gln Ala Tyr Leu Arg Gly Arg Lys
   1 5 10 15
<210> 43
   <211> 15
   <212> PRT
   <213> Artificial sequence
<220>
   <223> Synthetic peptide
<400> 43
   Thr Leu Gln Leu Leu Cys Leu Arg Ala Tyr Ile Lys Phe Cys Arg
   1 5 10 15
<210> 44
   <211> 15
   <212> PRT
   <213> Artificial sequence
<220>
   <223> Synthetic peptide
<400> 44
   Ser Leu Gln Cys Ile Ala Gly Gly Gln Val Leu Ala Ser Trp Phe
   1 5 10 15
<210> 45
   <211> 15
   <212> PRT
   <213> Artificial sequence
<220>
   <223> Synthetic peptide
<400> 45
   Ser Leu Gln Cys Met Ser Ala Gly Met Leu Leu Gly Arg Trp Phe
   1 5 10 15
<210> 46
   <211> 15
   <212> PRT
   <213> Artificial sequence
<220>
   <223> Synthetic peptide
<400> 46
   Ser Leu Gln Cys Met Ala Gly Gly Ala Val Leu Ala Val Trp Phe
   1 5 10 15
<210> 47
   <211> 15
   <212> PRT
   <213> Artificial sequence
<220>
   <223> Synthetic peptide
<400> 47
   Ser Leu Gln Cys Ile Ala Gly Gly Gln Val Leu Ala Ser Trp Phe
   1 5 10 15
<210> 48
   <211> 15
   <212> PRT
   <213> Artificial sequence
<220>
   <223> Synthetic peptide
<400> 48
   Ser Leu Gln Cys Arg Ala Gly Gly Thr Leu Leu Ala Val Trp Phe
   1 5 10 15
<210> 49
   <211> 15
   <212> PRT
   <213> Artificial sequence
<220>
   <223> Synthetic peptide
<400> 49
   Ser Leu Arg Cys Met Ala Gly Gly Ala Val Leu Ala Leu Trp Phe
   1 5 10 15
<210> 50
   <211> 15
   <212> PRT
   <213> Artificial sequence
<220>
   <223> Synthetic peptide
<400> 50
   Ser Leu Gln Cys Lys Ala Gly Gly Val Val Leu Ala Asn Trp Phe
   1               5                   10                  15
<210> 51
   <211> 15
   <212> PRT
   <213> Artificial sequence
<220>
   <223> Synthetic peptide
<400> 51
   Ser Leu Arg Cys Met Ala Gly Gly Ala Val Leu Ala Leu Trp Phe
   1               5                   10                  15
<210> 52
   <211> 15
   <212> PRT
   <213> Artificial sequence
<220>
   <223> Synthetic peptide
<400> 52
   Ser Leu Arg Cys Met Ala Gly Gly Ala Val Leu Ala Leu Trp Phe
   1               5                   10                  15
<210> 53
   <211> 15
   <212> PRT
   <213> Artificial sequence
<220>
   <223> Synthetic peptide
<400> 53
   Ser Leu Arg Cys Met Ala Gly Gly Ala Val Leu Ala Leu Trp Phe
   1               5                   10                  15
<210> 54
   <211> 15
   <212> PRT
   <213> Artificial sequence
<220>
   <223> Synthetic peptide
<400> 54
   Ser Leu Gln Cys Ile Ala Gly Gly Ala Val Leu Ala Ile Trp Phe
   1 5 10 15
<210> 55
   <211> 15
   <212> PRT
   <213> Artificial sequence
<220>
   <223> Synthetic peptide
<400> 55
   Ser Leu Gln Cys Leu Ser Ala Thr Gln Val Leu Lys Glu Phe Leu
   1 5 10 15
<210> 56
   <211> 15
   <212> PRT
   <213> Artificial sequence
<220>
   <223> Synthetic peptide
<400> 56
   Ser Leu Gln Cys Arg Ala Met Arg Arg Ile Leu Leu His Val Ile
   1 5 10 15
<210> 57
   <211> 15
   <212> PRT
   <213> Artificial sequence
<220>
   <223> Synthetic peptide
<400> 57
   Ser Leu Gln Cys Leu Ala Ala Lys Gln Val Leu Leu Lys Cys Phe
   1 5 10 15
<210> 58
   <211> 15
   <212> PRT
   <213> Artificial sequence
<220>
   <223> Synthetic peptide
<400> 58
   Ser Leu Gln Cys Leu Ala Ala Lys Ser Val Leu Leu Ser Cys Phe
   1 5 10 15
<210> 59
   <211> 17
   <212> PRT
   <213> Artificial sequence
<220>
   <223> Synthetic peptide
<400> 59
   Ser Leu Gln Tyr Leu Ala Leu Lys Ala Leu Val Thr Pro Lys Lys Ile
   1 5 10 15
   Lys
<210> 60
   <211> 17
   <212> PRT
   <213> Artificial sequence
<220>
   <223> Synthetic peptide
<400> 60
   Ser Leu Gln Tyr Leu Ala Leu Ala Ala Leu Ile Thr Pro Lys Lys Ile
   1 5 10 15
   Lys
<210> 61
   <211> 17
   <212> PRT
   <213> Artificial sequence
<220>
   <223> Synthetic peptide
<400> 61
   Ser Leu Gln Tyr Leu Ala Leu Thr Ala Leu Ile Lys Pro Lys Lys Ile
   1 5 10 15
   Lys
<210> 62
   <211> 17
   <212> PRT
   <213> Artificial sequence
<220>
   <223> Synthetic peptide
<400> 62
   Ser Leu Gln Tyr Leu Ala Leu Thr Ala Leu Ile Thr Pro Lys Lys Ile
   1 5 10 15
   Lys
<210> 63
   <211> 17
   <212> PRT
   <213> Artificial sequence
<220>
   <223> Synthetic peptide
<400> 63
   Ser Leu Gln Tyr Leu Ala Leu Lys Ala Leu Val Thr Pro Thr Arg Thr
   1 5 10 15
   Arg
<210> 64
   <211> 17
   <212> PRT
   <213> Artificial sequence
<220>
   <223> Synthetic peptide
<400> 64
   Ser Leu Gln Tyr Leu Ala Leu Thr Ala Leu Val Ala Pro Lys Lys Thr
   1 5 10 15
   Lys
<210> 65
   <211> 17
   <212> PRT
   <213> Artificial sequence
<220>
   <223> Synthetic peptide
<400> 65
   Thr Leu Gln Leu Leu Ala Leu Arg Ala Val Val Lys Ala Arg Ser Arg
   1 5 10 15
   Lys
<210> 66
   <211> 17
   <212> PRT
   <213> Artificial sequence
<220>
   <223> Synthetic peptide
<400> 66
   Thr Leu Gln Phe Leu Ala Leu Lys Ala Val Val Lys Val Lys Arg Asn
   1               5                   10                  15
   Lys
<210> 67
   <211> 17
   <212> PRT
   <213> Artificial sequence
<220>
   <223> Synthetic peptide
<400> 67
   Thr Leu Gln Tyr Leu Ala Leu Thr Ala Trp Val Gly Ala Lys Lys Arg
   1               5                   10                  15
   Lys
<210> 68
   <211> 17
   <212> PRT
   <213> Artificial sequence
<220>
   <223> Synthetic peptide
<400> 68
   Ser Leu Gln Phe Leu Ala Leu Lys Ala Leu Ile Ser Glu Arg Arg His
   1               5                   10                  15
   Arg
<210> 69
   <211> 17
   <212> PRT
   <213> Artificial sequence
<220>
   <223> Synthetic peptide
<400> 69
   Ser Leu Gln Phe Leu Ala Leu Lys Ala Leu Val Gly Gln Ser Lys Arg
   1 5 10 15
   Arg
<210> 70
   <211> 17
   <212> PRT
   <213> Artificial sequence
<220>
   <223> Synthetic peptide
<400> 70
   Ser Leu Gln Tyr Leu Ala Leu Arg Ala Trp Val Arg Val Gly Lys Lys
   1 5 10 15
   Lys
<210> 71
   <211> 15
   <212> PRT
   <213> Artificial sequence
<220>
   <223> Synthetic peptide
<400> 71
   Pro Leu Met Asp Leu Cys Arg Arg Ser Val Arg Leu Ala Leu Gly
   1 5 10 15
<210> 72
   <211> 15
   <212> PRT
   <213> Artificial sequence
<220>
   <223> Synthetic peptide
<400> 72
   Ser Leu Leu His Leu Ser Arg Leu Cys Val Arg His Asn Leu Gly
   1 5 10 15
<210> 73
   <211> 15
   <212> PRT
   <213> Artificial sequence
<220>
   <223> Synthetic peptide
<400> 73
   Pro Leu Met Asp Leu Cys Arg Arg Ser Ile Arg Ser Ala Leu Gly
   1 5 10 15
<210> 74
   <211> 15
   <212> PRT
   <213> Artificial sequence
<220>
   <223> Synthetic peptide
<400> 74
   Ser Leu Gln Asp Leu Cys Cys Arg Ala Ile Val Ser Cys Thr Pro
   1 5 10 15
<210> 75
   <211> 15
   <212> PRT
   <213> Artificial sequence
<220>
   <223> Synthetic peptide
<400> 75
   Ala Leu Phe Glu Leu Cys Gly Arg Ala Val Ser Ala His Met Gly
   1 5 10 15
<210> 76
   <211> 11
   <212> PRT
   <213> Artificial sequence
<220>
   <223> Synthetic peptide
<400> 76
   Ser Leu Gln Cys Ile Ala Gly Gly Gln Val Leu
   1 5 10
<210> 77
   <211> 11
   <212> PRT
   <213> Artificial sequence
<220>
   <223> Synthetic peptide
<400> 77
   Ser Leu Gln His Leu Cys Arg Leu Val Ile Asn
   1 5 10
<210> 78
   <211> 11
   <212> PRT
   <213> Artificial sequence
<220>
   <223> Synthetic peptide
<400> 78
   Ser Leu Asn Lys Met Cys Ser Asn Leu Leu Glu
   1 5 10
<210> 79
   <211> 14
   <212> PRT
   <213> Artificial sequence
<220>
   <223> Synthetic peptide
<400> 79
   Leu Phe Glu Leu Cys Gly Arg Ala Val Ser Ala His Met Gly
   1 5 10
<210> 80
   <211> 15
   <212> PRT
   <213> Artificial sequence
<220>
   <223> Synthetic peptide
<400> 80
   Ser Leu Gln His Leu Cys Arg Leu Val Ile Asn Arg Leu Val Ala
   1 5 10 15
<210> 81
   <211> 28
   <212> PRT
   <213> Artificial sequence
<220>
   <223> Synthetic peptide
<400> 81
   Lys Lys Arg Thr Leu Arg Lys Asn Asp Arg Lys Lys Arg Ser Leu Gln
   1 5 10 15
   Tyr Leu Cys Arg Phe Val Ile Arg Gln Tyr Thr Arg
   20 25
<210> 82
   <211> 28
   <212> PRT
   <213> Artificial sequence
<220>
   <223> Synthetic peptide
<400> 82
   Ser Leu Gln Tyr Leu Cys Arg Phe Val Ile Arg Gln Tyr Thr Arg Lys
   1               5                   10                  15
   Lys Arg Thr Leu Arg Lys Asn Asp Arg Lys Lys Arg
               20                  25
<210> 83
   <211> 28
   <212> PRT
   <213> Artificial sequence
<220>
   <223> Synthetic peptide
<400> 83
   Asn Leu Gln Asp Leu Cys Arg Ile Lys Ile Arg Gln Cys Ile Gly Lys
   1               5                   10                  15
   Lys Arg Thr Leu Arg Lys Asn Asp Arg Lys Lys Arg
               20                  25
<210> 84
   <211> 28
   <212> PRT
   <213> Artificial sequence
<220>
   <223> Synthetic peptide
<400> 84
   Ser Leu Gln His Leu Cys Arg Cys Ala Leu Arg Ser His Leu Glu Lys
   1               5                   10                  15
   Lys Arg Thr Leu Arg Lys Asn Asp Arg Lys Lys Arg
               20                  25
<210> 85
   <211> 28
   <212> PRT
   <213> Artificial sequence
<220>
   <223> Synthetic peptide
<400> 85
   Ser Leu Lys His Leu Cys Arg Leu Lys Ile Arg Lys Cys Met Gly Lys
   1               5                   10                  15
   Lys Arg Thr Leu Arg Lys Asn Asp Arg Lys Lys Arg
               20                  25

## Description

### TECHNICAL FIELD

The present invention relates to a method of transporting (inserting) a desired peptide motif of interest into the nucleus (typically the nucleolus) of a eukaryotic cell from outside of the cell, and to an artificial peptide used in such a method.

### BACKGROUND ART

Physiologically active substances such as polypeptides are sometimes inserted into, for example, human and other mammalian cells (eukaryocytes) so as to transform the characteristics of those cells (and tissues or organs composed of the cells) or to improve and increase the functions of the cells.
For example, Patent Document 1 discloses a cell-permeable carrier peptide for introducing a polypeptide, DNA or the like into cells. This patent document states that, by using a carrier peptide conjugate composed of a cell-permeable carrier peptide coupled with a different polypeptide, DNA or the like, physiologically active substances such as a polypeptide or DNA can be very efficiently inserted into cells.

As an alternative to methods which involve inserting into a target cell, as the physiologically active substance to be introduced, a polypeptide having a relatively large molecular weight so as to transform the characteristics and improve (or increase) the function of the cell, there also exist methods for introducing into the cell the portion of the amino acid sequence which is the smallest unit capable of expressing a specific function of interest possessed by the polypeptide, i.e., the amino acid sequence which constitutes a peptide motif.
For example, Patent Document 2 discloses that an amino acid sequence making up part or all of a specific region, known as the "BC-box," which is a portion of the peptide chain (amino acid sequence) making up SOCS proteins and family proteins thereof (referred to collectively below as "SOCS proteins") and is thought to bond to the Elongin BC complex, is a motif having a high neuronal differentiation-inducing activity in somatic stem cells. Patent Document 2 also discloses that, by introducing this motif into mammalian somatic stem cells, the resulting transduced cells can be induced to differentiate into neurons.
Patent Document 1: Japanese Patent No. 3854995
Patent Document 2: WO 2007/010989
Non-Patent Document 1: Journal of Biological Chemistry, Vol. 281, No. 35, pp. 25223-25230 (2006)
Non-Patent Document 2: PNAS, Vol. 95, pp. 114-119 (1998)
Non-Patent Document 3: Genes & Development, Vol. 12, pp. 3872-3881 (1998)
Non-Patent Document 4: Genes & Development, Vol. 18, pp. 2867-2872 (2004)
Non-Patent Document 5: Genes & Development, Vol. 18, pp. 3055-3065 (2004)
An important problem that must be addressed when introducing a peptide motif having some type of function like that mentioned above into a cell is the question of to which site (or organelle) within the cell should the motif of interest (and the peptide having that motif) be transported. This is because the degree of the effects conferred on the transduced cell is thought to differ markedly depending on the site to which transport takes place. For example, in cases involving the insertion of a motif having a function related to cell differentiation or transformation, as with the above-mentioned induction of neuronal differentiation, it is sometimes preferable to transport the motif into the nucleus rather than into the cytoplasm, and sometimes even more preferable to transport the motif into the nucleolus, which is where ribosome RNA synthesis takes place.
However, although conventional cell-permeable peptides like those mentioned in Patent Documents 1 and 2 (such as TAT, a protein transduction domain from HIV) are useful as tools for passing through a cell membrane from outside the cell and inserting a peptide motif of interest into the cytoplasm, they have a very limited transporting ability into the nucleus and moreover cannot be expected to transport a peptide motif of interest (peptide fragment) to the nucleolus.

### DISCLOSURE OF THE INVENTION

The present invention was conceived in order to resolve the foregoing problems in the prior art. Accordingly, it is an object of the invention to provide a method of inserting a desired peptide motif of interest into a eukaryotic cell from outside the cell (i.e., outside the cell membrane), and furthermore transporting the peptide motif at a high efficiency into the nucleus (preferably even to the nucleolus). Further objects of the invention are to provide an artificial peptide as a means for carrying out such a method, and a method of producing such an artificial peptide. A still further object of the invention is to provide a cell or a tissue which has acquired a function or characteristic by means of the method and artificial peptide provided by this invention.
One of the methods provided by the present invention is a method of transporting a peptide motif of interest into a nucleus (preferably a nucleolus) of a eukaryotic cell, such as a human or other mammalian cell, from outside the cell (i.e., from outside the cell membrane). The method disclosed herein includes: chemically synthesizing a peptide chain having an amino acid sequence constituting the peptide motif of interest at an N-terminal end or a C-terminal end of a "cell membrane-permeable nucleolar localization signal sequence" defined by the amino acid sequence KKRTLRKNDRKKR (SEQ ID NO: 1); and adding the chemically synthesized peptide to a culture medium which includes the eukaryotic cell or a tissue containing the eukaryotic cell. The method typically also includes culturing the eukaryotic cell or the eukaryotic cell-containing tissue, in the medium containing the chemically synthesized polypeptide and introducing the chemically synthesized polypeptide into the culturing eukaryotic cell by cell membrane permeability of the chemically synthesized polypeptide itself.
Here, "peptide motif (sequence motif)" is a term used in the present specification in the same sense as is commonly used in the field to which the invention pertains; this term denotes a partial structure serving as a functional minimum unit of a polypeptide (protein). That is, "peptide motif" refers to a relatively short amino acid sequence that can be associated with some function or structure. In this sense, the amino acid sequence shown in SEQ ID NO: 1 may also be understood to be a peptide motif which takes part in peptide transport into the nucleus (typically, the nucleolus) from outside the cell.

The inventors have discovered that when a peptide containing the amino acid sequence shown in SEQ ID NO: 1, which, as mentioned in Non-Patent Document 1, is known as a nucleolar localization signal (NoLS), and also containing another amino acid sequence which constitutes a peptide motif of interest (for specific examples, see the subsequently described examples) is synthesized, and the synthesized peptide is added to eukaryotic cells which are being cultured, this peptide is able to pass through the cell membrane of the target cells at a high efficiency and also is able to pass through the nuclear membrane at a high efficiency.
Accordingly, with the above-described inventive method, by constructing (synthesizing) an artificial peptide obtained through the combination of an amino acid sequence constituting a peptide motif of interest (i.e., a motif having a function that one wishes to introduce into a target cell) with the "cell membrane-permeable nucleolar localization signal sequence" defined by above SEQ ID NO: 1, and adding the artificial peptide to a target eukaryotic cell, the peptide motif of interest (i.e., an artificial peptide containing the motif) can be very efficiently transported into the nucleus (or the nucleolus) of the eurkaryotic cell from outside the cell (outside the cell membrane).

In a preferred embodiment of the peptide motif transporting method disclosed herein, a total number of amino acid residues constituting the synthesized peptide is not more than 500.
Such a peptide having a relatively short chain length (typically a linear (straight-chain) peptide) is desirable because it is easy to chemically synthesize and can be easily introduced into the target eukaryotic cell.

In another preferred embodiment of the peptide motif transporting method disclosed herein, the targeted eukaryotic cell to which the motif is introduced is a human stem cell or a non-human mammalian origin stem cell.
By transporting a motif of interest having a given function within human or non-human mammalian stem cells (e.g., somatic stem cells) into the nucleus (and more preferably the nucleolus), the present invention makes it possible to transform the stem cells, e.g., to differentiate the stem cells into specific cells (nerve cells, bone cells, muscle cells, skin cells, etc.).

To achieve the above objects, the present invention also provides a chemically synthesized polypeptide having cell membrane permeability which is used for the purpose of transporting a peptide motif of interest into the nucleus (preferably the nucleolus) of a eukaryotic cell from outside the cell.
This polypeptide is composed of a polypeptide chain synthesized so as to have, at an N-terminal end or a C-terminal end of a cell membrane-permeable nucleolar localization signal sequence defined by the amino acid sequence KKRTLRKNDRKKR (SEQ ID NO: 1), an amino acid sequence composed of a peptide motif which does not, in nature, exist next to the signal sequence, wherein the total number of amino acid residues constituting the synthesized polypeptide is not more than 500.
Through the use of a peptide having a simple sequence structure constructed by bringing into close proximity the above "cell membrane-permeable and nucleolar transport sequence" and the peptide motif of interest (sequence motif), the target motif of interest (amino acid sequence having a function) can be transported at a high efficiency into the cytoplasm, and moreover into the nucleus (preferably the nucleolus) from outside the cell membrane of the eukaryotic cell. An artificial peptide (typically, a linear peptide) having a total number of amino acid residues of not more than 500 is used because chemical synthesis is easy and such a peptide can be introduced at a high efficiency into a eukaryotic cell.

To achieve the above objects, the invention further provides a method of producing a chemically synthesized polypeptide having cell membrane permeability for transporting a peptide motif of interest into the nucleus (preferably, the nucleolus) of a eukaryotic cell from outside the cell. This production method includes: selecting the peptide motif of interest and an amino acid sequence constituting the motif; designing a peptide chain having an amino acid sequence constituting the selected peptide motif at an N-terminal end or a C-terminal end of a cell membrane-permeable nucleolar localization signal sequence defined by the amino acid sequence KKRTLRKNDRKKR (SEQ ID NO: 1); and chemically synthesizing the designed peptide chain.
The peptide chain is designed in such a manner that a total number of amino acid residues is not more than 500.

### TEXT IN SEQUENCE LISTING

### SEQ ID NOS: 1 to 85: Synthetic peptides

### BRIEF DESCRIPTION OF THE DRAWINGS

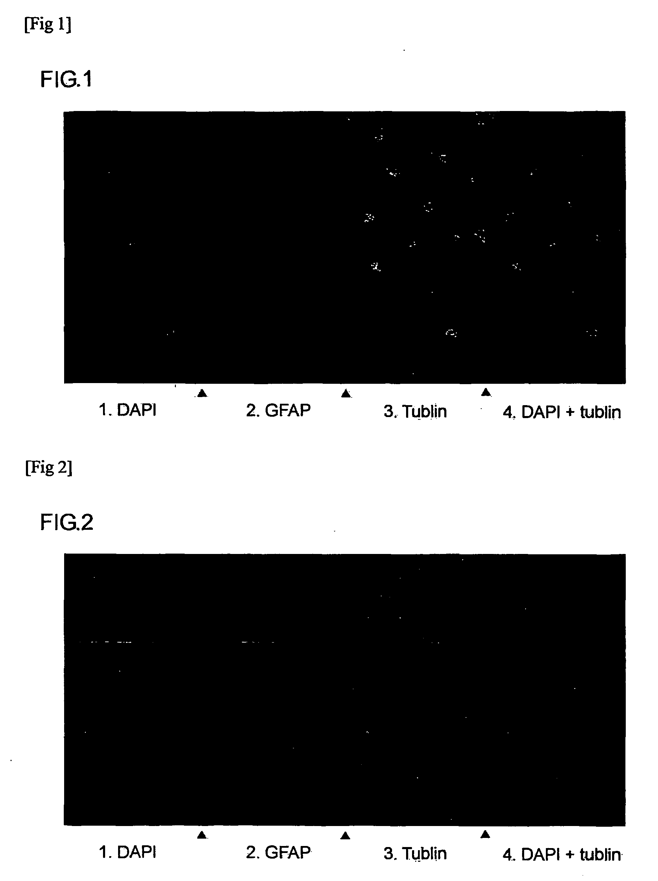

FIG. 1 shows fluorescence micrographs (images) showing the state of neuronal differentiation in cultured cells prepared by adding the peptide of Sample 2 to a culture broth of rat neural stem cells to a broth concentration of 1 µM, and culturing the cells for 24 hours; the micrograph in FIG. 1 being divided equally into four areas; the first area from the left side showing a plot that arose by nuclear staining with DAPI; the second area from the left side showing a fluorescent state due to the presence of fluorescent dye-labeled anti-GFAP antibody; the third area from the left side showing a fluorescent state due to the presence of fluorescent dye-labeled anti-tubulin antibody; and the fourth area (right-most) from the left side showing, superimposed on each other, the plot that arose by nuclear staining with DAPI and the fluorescent state due to the presence of fluorescent dye-labeled anti-tubulin antibody.
FIG. 2 shows fluorescence micrographs (images) showing the state of neuronal differentiation in cultured cells prepared by adding the peptide of Comparative Sample 1 to a culture broth of rat neural stem cells to a broth concentration of 1 µM, and culturing the cells for 24 hours; the micrograph in FIG. 2 being divided equally into four areas; the first area from the left side showing a plot that arose by nuclear staining with DAPI; the second area from the left side showing a fluorescent state due to the presence of fluorescent dye-labeled anti-GFAP antibody; the third area from the left side showing a fluorescent state due to the presence of fluorescent dye-labeled anti-tubulin antibody; and the fourth area (right-most) from the left side showing, superimposed on each other, the plot that arose by nuclear staining with DAPI and the fluorescent state due to the presence of fluorescent dye-labeled anti-tubulin antibody.
FIG. 3 is a micrograph obtained using a confocal laser scanning microscope to observe specimens (cells) that were methanol fixed after a cell suspension to which had been added a FITC-labeled artificial peptide according to an example of the invention was cultured for 0.5 hour.
FIG. 4 is a micrograph obtained using a confocal laser scanning microscope to observe specimens (cells) that were methanol fixed after a cell suspension to which had been added a FITC-labeled artificial peptide according to an example of the invention was cultured for 1 hour.
FIG. 5 is a micrograph obtained using a confocal laser scanning microscope to observe specimens (cells) that were methanol fixed after a cell suspension to which had been added a FITC-labeled artificial peptide according to an example of the invention was cultured for 2 hours.
FIG. 6 is a micrograph obtained using a confocal laser scanning microscope to observe specimens (cells) that were methanol fixed after a cell suspension to which had been added a FITC-labeled artificial peptide according to an example of the invention was cultured for 6 hours.

### BEST MODE FOR CARRYING OUT THE INVENTION

Following is a detailed description of preferred embodiments of the present invention. Note that technical matters other than those matters particularly mentioned in the present specification (e.g., the primary structure and chain length of an artificial peptide that has been constructed) which are required for carrying out the present invention (e.g., general matters such as relate to peptide synthesis, polynucleotide synthesis, and cell cultivation) are matters of design variation that could be apprehended by a person skilled in the art based on prior art in such fields as medicine, pharmacology, organic chemistry, biochemistry, genetic engineering, protein engineering, molecular biology and hygieiology.

The present invention can be practiced based on the technical details disclosed in the present specification and on common general technical knowledge in the pertinent fields. In the following description, amino acids are indicated by single-letter designations (in sequence listings, by three-letter designations) in accordance with the nomenclature for amino acids set forth in the IUPAC-IUB guidelines.

In the present specification, "artificially synthesized artificial peptide" refers to a peptide chain which does not by itself independently exist stably in the natural world, and is instead a synthetic peptide manufactured by artificial chemical synthesis or biosynthesis (i.e., genetic engineering-based production).

In this specification, "peptide" is a term which denotes an amino acid polymer having a plurality of peptide bonds, and is not limited by the number of amino acid residues included on the peptide chain. Therefore, both oligopeptides having up to about ten amino acid residues and polypeptides composed of a greater number of amino acid residues than this are encompassed by the term 'artificial peptide' in this specification.

As used herein, unless specified otherwise, "amino acid residue" is a term which includes the N-terminal amino acid and the C-terminal amino acid of a peptide chain.

In this specification, "polynucleotide" is a term denoting a polymer (nucleic acid) in which a plurality of nucleotides are linked by phosphodiester bonds, and is not limited by the number of nucleotides. As used herein, the term 'polynucleotide' encompasses DNA fragments and RNA fragments of various lengths.

The "cell membrane-permeable nucleolar localization signal sequence" disclosed herein is a sequence defined by (understood as) the amino acid sequence of SEQ ID NO: 1, and is characterized by being an amino acid sequence (signal sequence) which exhibits cell membrane permeability and nuclear transportability (nuclear membrane permeability).

The specific amino acid sequence indicated here in SEQ ID NO: 1 is a sequence which, in addition to being a nucleolar localization signal corresponding to a sequence portion (i.e., a motif) composed of the total of 13 amino acid residues from the amino acid residue at position 491 to the amino acid residue at position 503 of LIM Kinase 2 (see Non-Patent Document 1) within human endothelial cells, which is a type of protein kinase that takes part in intracellular signal transmission, is a sequence that was discovered by the inventors to exhibit an excellent cell membrane permeability.

Therefore, the "cell membrane-permeable nucleolar localization signal sequence" disclosed herein and defined by (understood as) the amino acid sequence of SEQ ID NO: 1 is a sequence which is typically identical to the amino acid sequence of SEQ ID NO: 1, but which, aside from such an identical sequence, also encompasses amino acid sequences formed by the replacement, deletion and/or addition (insertion) of one or a plurality (e.g., up to five, and typically two or three) of amino acid residues without a loss in the cell membrane permeability and nuclear transportability. The reason is that such minimally modified sequences are easy to use by persons of ordinary skill in the art based on the information disclosed herein, and are encompassed by the technical concept disclosed herein of a "cell membrane-permeable nucleolar localization signal sequence." Typical examples include sequences that arise from conservative amino acid replacement involving the conservative replacement of one or a plurality (typically two or three) of the amino acid residues on the amino acid sequence in SEQ ID NO: 1 (e.g., sequences in which a basic amino acid sequence has been replaced with another basic amino acid residue), or sequences in which one or a plurality (typically, two or three) of the amino acid residues on a given amino acid sequence have been added (inserted) or deleted.

The artificial peptide used in the peptide motif transporting method disclosed herein is a peptide which can be designed and built by linking the amino acid sequence constituting the desired peptide motif (which term also encompasses herein a domain) to an N-terminal end and/or a C-terminal end of the above cell membrane-permeable nucleolar localization signal sequence. The motif used in building a single artificial peptide is not limited to one motif. For example, the peptide chain may be designed so that two or more units of one type of peptide motif (amino acid sequence) are present (e.g., with the two or more units arranged so as to be connected in tandem), and the peptide chain may be designed using a plurality of peptide motifs of different types (i.e., different functions) or peptide motifs of the same function but mutually differing sequences.

The same applies also to the cell membrane-permeable nucleolar localization signal sequence. Namely, although it suffices for at least one unit of cell membrane-permeable nucleolar localization signal sequence to be present on a single peptide chain, the invention is not limited in this regard; i.e., a plurality of cell membrane-permeable nucleolar localization signal sequences, either of the same sequence or of mutually differing sequences, may be present at a plurality of places on a single peptide chain.

The motif (domain) used in artificial peptide construction is not subject to any particular limitation; various hitherto known sequence motifs may be used. For example, when the eukaryotic cell targeted for insertion of the peptide motif is a human or other mammalian stem cell (inclusive of somatic stem cells, embryonic stem cells, artificial pluripotent stem cells), the use of various motifs which take part in inducing the differentiation of such stem cells is preferred. When the eukaryotic cell targeted for insertion is a cancer cell (tumor cell), the use of various motifs which take part in inducing apoptosis of the cancer cell (tumor cell) is preferred.

Preferred examples of peptide motifs that may be particularly advantageously used to practice the invention include the various amino acid sequences (motifs) capable of exhibiting an ability to induce neuronal differentiation which are mentioned in Patent Document 2.

That is, Patent Document 2 mentions that the amino acid sequences which are included in specific regions known as a "BC-boxes" that are portions of the amino acid sequences making up SOCS proteins (cytokine information transmission suppressors) having a SOCS-box, a region (amino acid sequence) capable of bonding to the Elongin BC complex (specifically, a portion of Elongin C) that is known to form a complex with Elongin A and act as a transcriptional regulatory factor, and family proteins thereof (referred to collectively below as "SOCS proteins"), and which are thought to bond to the Elongin BC complex, have a high neuronal differentiation-inducing activity in somatic stem cells.

SEQ ID NOS: 2 to 19, which are typical examples thereof, are amino acid sequences included in various protein BC-boxes which have been identified as SOCS proteins (see Non-Patent Documents 2 to 5).

Illustrative examples include the amino acid sequences composed of 15 consecutive amino acid residues from the N-terminus of the BC-box which are included in mSOCS-1 (SEQ ID NO: 2), mSOCS-2 (SEQ ID NO: 3), mSOCS-3 (SEQ ID NO: 4), mSOCS-4 (SEQ ID NO: 5), mSOCS-5 (SEQ ID NO: 6), hSOCS-6 (SEQ ID NO: 7), hSOCS-7 (SEQ ID NO: 8), hRAR-1 (SEQ ID NO: 9), hRAR-like (SEQ ID NO: 10), mWSB-1 (SEQ ID NO: 11), mWSB-2 (SEQ ID NO: 12), mASB-1 (SEQ ID NO: 13), mASB-2 (SEQ ID NO: 14), hASB-3 (SEQ ID NO: 15), LRR-1 (SEQ ID NO: 16), hASB-7 (SEQ ID NO: 17), mASB-10 (SEQ ID NO: 18), and hASB-14 (SEQ ID NO: 19) (see Non-Patent Documents 2 to 5).

Although a detailed explanation is omitted here, SEQ ID NOS: 20 to 80 show the amino acid sequences included in the BC-boxes of various SOCS proteins identified from viruses (e.g., HIV, AdV, SIV) and mammals, and the peptides composed of those sequences. For example, SEQ ID NO: 75 and SEQ ID NO: 79 are amino acid sequences included in the BC-box of a SOCS protein (MUF1) identified from man. SEQ ID NO: 80 is an amino acid sequence included in the BC-box of the SOCS protein mCIS (cytokine-inducible SH2-containing protein) identified from the mouse.

These examples are illustrative only, there being no intention here to limit the amino acid sequences (motifs) of the BC-boxes to those mentioned above. Even if not mentioned here, the constituent amino acid sequences of various BC-boxes are mentioned in numerous documents that were already published at the time this application was filed. Such amino acid sequences are easily knowable by ordinary search means.

In a preferred embodiment of the present invention, any of the above-mentioned amino acid sequences from a BC-box (typically, any of the amino acid sequences from among SEQ ID NOS: 2 to 80) may be used as a peptide motif (sequence motif) which takes part in the induction of neuronal differentiation, and is capable of building an artificial peptide for insertion into a target eukaryotic cell (e.g., a somatic stem cell of human or mammalian origin). Therefore, as is apparent from the above explanation, the present invention provides a method which induces the differentiation of at least one type of eukaryotic cell into nerve cells. That is, this method includes: synthesizing a peptide chain having, at a N-terminal end or a C-terminal end of the cell membrane-permeable nucleolar localization signal sequence of the invention, an amino acid sequence from a BC-box as the peptide motif which participates in the induction of neuronal differentiation (typically an amino acid sequence composed of at least ten (e.g., at least ten from the N-terminus) consecutive amino acid residues selected from the amino acid sequences shown in any of SEQ ID NOS: 2 to 80); and adding the synthesized peptide (artificial peptide) to a culture medium or living organism which includes the target eukaryotic cell or a tissue containing the eukaryotic cell. Typically, the method further includes culturing the eukaryotic cell to which the synthesized peptide is added, or the tissue containing the cell.

In addition, as is apparent from the above explanation, the present description also discloses an artificial peptide for use in a method of inducing differentiation in such nerve cells, and a method of producing such an artificial peptide.
That is, the artificial peptide (neuronal differentiation-inducing peptide) composed in this way is synthesized so as to have an amino acid sequence from a BC-box as the peptide motif associated with the induction of neuronal differentiation (also referred to below as a "BC-box-related sequence"; typically an amino acid sequence composed of, at a N-terminal end or a C-terminal end of the above-mentioned cell membrane-permeable nucleolar localization signal sequence, at least ten (e.g., at least ten from the N-terminus) consecutive amino acid residues selected from among the amino acid sequences shown in any of SEQ ID NOS: 2 to 80). The total number of amino acid residues is preferably 50 or less.
As in the case of the above-described cell membrane-permeable nucleolar localization signal sequence, to the extent that the function as a peptide motif associated with the induction of neuronal differentiation is maintained, modified amino acid sequences obtained by the replacement, deletion and/or addition (insertion) of one or a plurality (e.g., up to five, and typically two or three) amino acid residues are also encompassed by the above "amino acid sequence from a BC-box (BC-box-related sequence)".

The neuronal differentiation-inducing peptide constituted as described above has a high neuronal differentiation-inducing activity on at least one type of cell (typically, a stem cell). For this reason, it can be advantageously used as an active ingredient in a neuronal differentiation inducer. The neuronal differentiation-inducing peptide included in the neuronal differentiation inducer, to the extent that there is no loss in the neuronal differentiation-inducing activity, may be in the form of a salt. For example, use may be made of an acid addition salt of the peptide, which may be obtained by subjecting a commonly used inorganic acid or an organic acid to an addition reaction according to a conventional method. Alternatively, to the extent that they have neuronal differentiation-inducing activities, use may be made of other salts (e.g., metal salts).

The neuronal differentiation inducer may also include, apart from the above-constituted neuronal differentiation-inducing peptide serving as the active ingredient, various carriers that are medically (pharmaceutically) acceptable for the mode of use. Carriers that are generally used in peptide medications as diluents, excipients or the like are preferred. Although these may suitably differ according to the use and form of the neuronal differentiating inducer, typical examples include water, physiological buffers and various organic solvents. The carrier may be an aqueous solution containing a suitable concentration of an alcohol (e.g., ethanol), glycerol, or a non-drying oil such as olive oil. Alternatively, the carrier may be liposomes. Examples of secondary ingredients that may be included in the neuronal differentiation inducer include various fillers, thickeners, binders, wetting agents, surfactants, dyes and fragrances.

The form of the neuronal differentiating inducer is not subject to any particular limitation. Examples of typical forms include liquid preparations, suspensions, emulsions, aerosols, foams, pellets powders, tablets, capsules and ointments. For use in injection or the like, the neuronal differentiating inducer may be rendered into a freeze-dried form or granules for preparing a drug solution by dissolution in physiological saline or a suitable buffer (e.g., PBS) just prior to use.

The process of preparing a drug (composition) in various forms by using as the materials the neuronal differentiation-inducing peptide (main ingredient) and various carriers (secondary ingredients) may itself be in general accordance with a conventional known method. Because such a preparation process itself is not distinctive to the present invention, a detailed description is omitted here. An example of a detailed information source relating to formulation is Comprehensive Medicinal Chemistry, edited by Corwin Hansch and published by Pergamon Press (1990).

The neuronal differentiation inducer may be used in a manner and dose that accords with the form thereof and the intended purpose.
For example, the neuronal differentiation-inducing peptide containing the BC-box-related sequence disclosed herein (i.e., the neuronal differentiation inducer containing this peptide) may be administered as a liquid preparation to the patient (i.e., in vivo) in exactly the desired amount by intravenous, intramuscular, hypodermal, intradermal or intraperitoneal injection. Alternatively, this neuronal differentiation-inducing peptide may be administered orally in a solid form such as tablets. In this way, nerve cells can be generated (produced) from somatic stem cells present within the living organism, typically at or near the site of disease. This makes it possible to effectively treat various neurological disorders for which nerve regeneration is an important mode of treatment. For example, the treatment of neurological disorders such as Parkinson disease, cerebral infarction, Alzheimer disease, paralysis of the body due to spinal cord injury, cerebral contusions, amyotrophic lateral sclerosis, Huntington disease, brain tumors and retinal degeneration by a regenerative medical approach is achieved.

Alternatively, by administering a suitable amount of a neuronal differentiation inducer (neuronal differentiation-inducing peptide) to a cellular material temporarily or permanently removed from a living organism, that is, to living tissue or a cell mass (e.g., a somatic stem cell culture), a peptide motif of interest (BC-box-related sequence) can be transported into the nucleus (preferably the nucleolus), enabling nerve cells to be efficiently generated. This means that the desired nerve cells can be produced in a large quantity within such cellular material.

Moreover, even when the nerve cells that have been produced in a large quantity, or cellular material (living tissue or cell mass) containing these produced nerve cells, are returned again to the living organism (typically, at the site of disease where neuronal regeneration is required), therapeutic effects similar to those obtained when a neuronal differentiation inducer (neuronal differentiation-inducing peptide) is administered directly in vivo are achievable.

As is apparent from the above explanation, this invention is also able to provide cells, cell masses or living tissue in which differentiation to nerve cells useful in the treatment of neurological disorders has been induced by using one of the above-constituted neuronal differentiation-inducing peptides disclosed herein.

Also, polynucleotides coding for the neuronal differentiation-inducing peptides of the invention may be used as materials employed in so-called gene therapy. For example, by integrating a gene (typically, a DNA segment or a RNA segment) coding for a neuronal differentiation-inducing peptide into a suitable vector and inserting the vector at the target site, it is possible to continuously express the neuronal differentiation-inducing peptide of the present invention within a living organism (cell). Therefore, polynucleotides (e.g., DNA segments, RNA segments) coding for the neuronal differentiation-inducing peptides of the present invention are useful as drugs for treating or preventing neurological disorders in the above types ofpatients.

In the artificial peptides for transporting peptide motifs provided by the present invention, such as the neuronal differentiation-inducing peptides described above as typical examples, at least one amino acid residue may be amidated. By amidating the carboxyl group on an amino acid residue (typically, the C-terminal amino acid residue of a peptide chain), the structural stability (e.g., the protease resistance) of the peptide within the cytoplasm and within the nucleus can be enhanced.

It is desirable for the artificial peptide to be a peptide in which the total number of amino acid residues making up the peptide chain is not more than 500 (preferably not more than 300, and most preferably not more than 100, such as 50 or less). Such peptides having a short chain length can be easily constructed by chemical synthesis techniques.

No particular limitation is imposed on the conformation of the peptide, although a linear or helical conformation is preferred from the standpoint if not readily becoming an immunogen (antigen).

The artificial peptide of the present invention is preferably a peptide in which all the amino acid residues are L-type amino acids. However, to the extent that there is no loss in the desired functions of the cell membrane-permeable nucleolar localization signal sequence and the peptide motif present therein, a peptide in which some or all of the amino acid residues have been replaced with D-type amino acids is also acceptable.

Also, to the extent that there is no loss in the desired functions of the cell membrane-permeable nucleolar localization signal sequence and peptide motif present therein, additional sequences which cannot be included in these sequences may be included in part.

Of the artificial peptides used, those having a relatively short peptide chain can be easily produced by an ordinary chemical synthesis process. For example, a conventional known solid-phase synthesis process or liquid-phase synthesis process may be used. A solid-phase synthesis process which employs t-butyloxycarbonyl (Boc) or 9-fluorenylmethoxycarbonyl (Fmoc) as a protective group on the amino group is preferred. That is, by means of a solid-phase synthesis process using a commercial peptide synthesizer (e.g., available from PerSeptive Biosystems, Applied Biosystems, etc.), it is possible to synthesize a peptide chain having the desired amino acid sequence and modifying (e.g., C-terminal amidating) portions.

Alternatively, the artificial peptide may be biosynthesized based on a genetic engineering technique. This approach is preferred in cases where a polypeptide having a relatively long peptide chain is produced. That is, the DNA of a nucleotide sequence (including the ATG initiation codon) which codes for the amino acid sequence of the desired artificial peptide is synthesized. Then, a recombinant vector having an expression gene construct composed of this DNA and various regulatory elements (including promoters, ribosome binding sites, terminators, enhancers, and various cis-elements which control the expression level) for expressing this amino acid sequence within a host cell is constructed in accordance with the host cell.

Using an ordinary technique, this recombinant vector is inserted into given host cells (e.g., yeast, insect cells, plant cells, animal (mammalian) cells), and the host cells or tissue or individuals containing those cells are cultured under specific conditions. In this way, the target polypeptide can be expressed and produced intracellularly. Next, by isolating from the host cells (when the polypeptide is secreted, from within the culture medium) and purifying the polypeptide, a peptide having the target amino acid sequence can be obtained. Using an ordinary technique, this recombinant vector is inserted into given host cells (e.g., yeasts, insect cells, plants cells, mammalian cells), and the host cells or tissue or individuals containing these cells are cultured under specific conditions. In this way, the target polypeptide can be expressed and produced intracellularly. Next, by isolating from the host cells (when the polypeptide is secreted, from the culture medium) and purifying the polypeptide, the target peptide can be obtained.

Methods hitherto used in the art may be directly employed without modification as the method for constructing the recombinant vector and the method for introducing the constructed recombinant vector into the host cell. Because such methods themselves are not distinctive to the present invention, detailed descriptions are omitted here.

For example, a fused protein expression system may be employed for efficient large-volume production within host cells. That is, a gene (DNA) coding for the amino acid sequence of the target peptide is chemically synthesized, and the synthesized gene is introduced to a preferred site on a suitable fused protein expression vector (a glutathione S-transferase (GST) fused protein expression vector such as the pET series available from Novagen and the pGEX series available from Amersham Bioscience). The host cells (typically, *Escherichia coli*) are then transformed by the vector. The resulting transformant is cultured, thereby producing the target fused protein. This protein is then extracted and purified. Next, the purified fused protein thus obtained is cleaved with a specific enzyme (protease), and the liberated target peptide fragments (the designed artificial peptide) are recovered by a method such as affinity chromatography. The target peptide may be produced by using such a conventional, known fused protein expression system (e.g., the GST/His system available from Amersham Bioscience may be used).

Alternatively, the target polypeptide may be synthesized in vitro by constructing template DNA for a cell-free protein synthesis system (i.e., a synthesized gene fragment having a nucleotide sequence which codes for the amino acid sequence of the target artificial peptide) and, using the various compounds required for peptide synthesis (e.g., ATP, RNA polymerase, amino acids), employing a cell-free protein synthesis system. For information concerning cell-free protein synthesis systems, reference may be made to, for example, Shimizu et al., Nature Biotechnology, 19, 751-755 (2001), and Madin et al., Proc. Natl. Acad. Sci. USA, 97(2), 559-564 (2000). Based on the technology described in these articles, many corporations had already carried out the commissioned production of polypeptides at the time this application was filed. Also, PROTEIOS™, a wheat germ cell-free protein synthesis kit available from Toyobo Co., Ltd. (Japan), is commercially available.

Therefore, so long as the amino acid sequence (e.g., the above-mentioned BC-box-related sequence) for a peptide motif to be introduced into the nucleus (preferably the nucleolus) has been selected and the peptide chain has been designed together with the above cell membrane-permeable nucleolar localization signal sequence, the target peptide can be easily synthesized and produced by a cell-free protein synthesis system in accordance with the amino acid sequence. For example, a peptide can be easily produced based on the Puresystem® from Post Genome Institute Co., Ltd, Japan.

Several examples of the invention are described below, although these examples are not intended to limit the scope of the invention.

### Example 1: Peptide Synthesis

A total of six types of peptides (Samples 1 to 5, Comparative Sample 1) were produced using the subsequently described peptide synthesizer. Table 1 shows the amino acid sequences of these synthesized peptides.

**Table 1**

| Sample No. | Amino acid sequence | | Total number of amino acid residues |
|---|---|---|---|
| Sample 1 | KKRTLRKNDRKKR-SLQYLCRFVIRQYTR | (SEQ ID NO: 81) | 28 |
| Sample 2 | SLQYLCRFVIRQYTR-KKRTLRKNDRKKR | (SEQ ID NO: 82) | 28 |
| Sample 3 | NLQDLCRIKIRQCIG-KKRTLRKNDRKKR | (SEQ ID NO: 83) | 28 |
| Sample 4 | SLQHLCRCALRSHLE-KKRTLRKNDRKKR | (SEQ ID NO: 84) | 28 |
| Sample 5 | SLKHLCRLKIRKCMG-KKRTLRKNDRKKR | (SEQ ID NO: 85) | 28 |
| Comparative Sample 1 | KKRTLRKNDRKKR | (SEQ ID NO: 1) | 13 |

As shown in Table 1, Samples 1 to 5 each have, at the N-terminal end or a C-terminal end thereof, a "cell membrane-permeable nucleolar localization signal sequence" composed of the amino acid sequence (13 amino acid residues) denoted as SEQ ID NO: 1. In addition, Samples 1 to 5 are constructed so as to have, as the peptide motif next to this cell membrane-permeable nucleolar localization signal sequence, a "BC-box-related sequence" which participates in the induction of neuronal differentiation.

Sample 1 is a synthesized peptide which has, at the C-terminal end of the cell membrane-permeable nucleolar localization signal sequence, the amino acid sequence of hSOCS-6 (SEQ ID NO: 7), and in which the total number of amino acid residues is 28 (SEQ ID NO: 81).

Sample 2 is a synthesized peptide which has, at the N-terminal end of the cell membrane-permeable nucleolar localization signal sequence, the amino acid sequence of hSOCS-6 (SEQ ID NO: 7), and in which the total number of amino acid residues is 28 (SEQ ID NO: 82).

Sample 3 is a synthesized peptide which has, at the N-terminal end of the cell membrane-permeable nucleolar localization signal sequence, the amino acid sequence of hASB-7 (SEQ ID NO: 17), and in which the total number of amino acid residues is 28 (SEQ ID NO: 83).

Sample 4 is a synthesized peptide which has, at the N-terminal end of the cell membrane-permeable nucleolar localization signal sequence, the amino acid sequence of mASB-10 (SEQ ID NO: 18), and in which the total number of amino acid residues is 28 (SEQ ID NO: 84).

Sample 5 is a synthesized peptide which has, at the N-terminal end of the cell membrane-permeable nucleolar localization signal sequence, the amino acid sequence of hASB-14 (SEQ ID NO: 19), and in which the total number of amino acid residues is 28 (SEQ ID NO: 85).

Comparative Sample 1 is a synthesized peptide which is made up only of the cell membrane-permeable nucleolar localization signal sequence and in which the total number of amino acid residues is 13 (SEQ ID NO: 1).

In all of these samples, the carboxyl group (-COOH) on the C-terminal amino acid has been amidated (-CONH₂).

Each of the above peptides was synthesized by a solid-phase synthesis process (Fmoc process) using a commercial peptide synthesizer (433A, a product of Applied Biosystems). HATU (a product of Applied Biosystems) was used as the condensing agent, and the resin and amino acid used in the solid-phase synthesis process were procured from NOVA Biochem. When the C-terminus of the amino acid sequence was amidated, Rink Amide resin (100 to 200 mesh) was used as the solid-phase support.

The deprotection reaction and condensation reaction were repeatedly carried out in accordance with the synthesis program of the peptide synthesizer, thereby elongating the peptide from the Fmoc-amino acid which bonds to the resin so as to obtain a synthesized peptide of the intended chain length. Specifically, the operations of cleaving and removing the Fmoc group serving as the amino protecting group on the amino acid with 20% piperidine/dimethylformamide (DMF) (peptide synthesis grade, a product of Kanto Chemical Co., Inc.), washing with DMF, reacting with 4 equivalents each of Fmoc-amino acid (-OH) and washing with DMF were repeated. After the peptide chain elongation reactions were entirely completed, the Fmoc group was cleaved with 20% piperidine/DMF, and the reaction product was washed, first with DMF, then with methanol.

Following solid-phase synthesis, the synthesized peptide chain was transferred together with the resin to a centrifuge tube, 1.8 mL of ethanediol, 0.6 mL of m-cresol, 3.6 mL of thioanisole and 24 mL of trifluoroacetic acid were added, and the mixture was stirred at room temperature for 2 hours. The resin that had been bonded to the peptide chain was then removed by filtration.

Next, cold ethanol was added to the filtrate and cooling was carried out with ice-cooled water to give a peptide precipitate. The supernatant was then discarded by centrifugal separation (5 minutes at 2500 rpm). Cold diethyl ether was subsequently added to the precipitate, which was thoroughly stirred, following which centrifugal separation was carried out under the same conditions as above. This stirring and centrifugal separation treatment were repeated a total of three times.

The resulting peptide precipitate was dried in vacuo, and purification was carried out using a high-performance liquid chromatograph (Waters 600, a product of Waters Corporation).

Specifically, using a precolumn (Guard-Pak Delta-pak C18 A300, a product of Nihon Waters K.K.) and a C18 reversed-phase column (XTerra® column, a product of Nihon Waters K.K.; MS C 18, 5 µm, 4.6x 150 mm), a mixture of 0.1% trifluoroacetic acid in water and 0.1 % trifluoroacetic acid in acetonitrile was used as the eluant. That is, while increasing over time the amount of the above acetonitrile solution of trifluoroacetic acid included in the eluant (in terms of the volumetric ratio, providing a concentration gradient of from 10% to 80%), separation and purification were carried out for 30 to 40 minutes using the above column at a flow rate of 1.5 mL/min. The peptide which eluted from the reversed-phase column was detected at a wavelength of 220 nm using an ultraviolet detector (490E Detector, a product of Waters Corporation), and indicated as a peak on a recording chart.

In addition, the molecular weights of each of the eluted peptides were determined based on matrix-assisted laser desorption time of flight mass spectrometry (MALDI-TOF/MS) using the Voyager DE RP™ manufactured by PerSeptive Biosystems. As a result, the target peptide was confirmed to have been synthesized and purified.

### Example 2: Evaluation of Neuronal Differentiation-Inducing Activity of Synthesized Artificial Peptide

The neuronal differentiation-inducing activities of the six artificial peptides obtained in Example 1 were examined.

That is, these sample peptides were added to a culture broth of neural stem cells collected from a rat, and incubated. The amount of peptide added was adjusted so that the peptide concentration within the culture broth became 1 µM for each peptide.

After 24 hours had elapsed following peptide addition, each sample was subjected to nuclear staining with 4',6-diamidino-2-phenylindole (DAPI), then examined under a fluorescence microscope.

In addition, the same sample was subjected to evaluation with a neuronal differentiation induction marker. That is, using tubulin as a marker for identifying neurons, the presence or absence of tubulin (i.e., of neurons) in the culture broth was determined by a fluorescent antibody method that uses a fluorescent dye-labeled anti-tubulin antibody for identifying this tubulin. In addition, using glial fibrillary acidic protein (GFAP), which is a cytoskeletal protein in glial cells, as a marker for identifying glial cells, the presence or absence of GFAP (i.e., of glial cells) in the culture broth was determined by a fluorescent antibody method that uses a fluorescent dye-labeled anti-GFAP antibody for identifying this GFAP.

As a result of the above tests, pronounced neuronal differentiation was confirmed when the artificial peptides (neuronal differentiation-inducing peptides) of Samples 1 to 5 were added. That is, as shown in FIG. 1, which are fluorescence micrographs (images) of cell cultures to which the peptide of Sample 2 had been added, a plot that arose from nuclear staining with DAPI (see, in FIG. 1 which has been divided equally into four areas, the first area from the left side) was observed; that is, fluorescence due to the presence of fluorescent dye-labeled anti-tubulin antibody was observed at more than 90% of the positions where nuclei (cells) are present (see, in FIG. 1 which has been divided equally into four areas, the third and fourth (right side) areas from the left side). This indicates that more than 90% of the surviving cells within the cell culture differentiated to neurons from the neural stem cells. Moreover, fluorescence due to the presence of fluorescent dye-labeled anti-GFAP antibody was not observed (see, in FIG. 1 which has been divided equally into four areas, the second area from the left side). Accordingly, differentiation to glial cells was not observed.

Meanwhile, as shown in FIG. 2, with regard to a cell culture in which the peptide of Comparative Sample 1 had been added, neither fluorescence due to the presence of fluorescent dye-labeled anti-tubulin antibody nor fluorescence due to the presence of fluorescent dye-labeled anti-GFAP antibody was observed. Hence, differentiation from neural stem cells to neurons (and glial cells) was not observed.

### Example 3: Evaluation of Cell Membrane-Permeability of Synthesized Artificial Peptides

The cell membrane permeabilities of the five artificial peptides having neuronal differentiation-inducing activities (see Example 2) obtained in Example 1 were investigated.

Specifically, striate bodies of the cerebellum collected from a mouse embryo (E14.5) were suspended in a growth medium for mouse neural stem cells (product of Cell Applications) and, by removing the tissue specimen through a cell strainer, a cell suspension containing mouse neural stem cells (mNSC) was prepared. Next, a cell suspension prepared in the above growth medium to a cell concentration of 2×10⁵ cells/mL was cultured for one week at 37°C and under 5% CO₂ in an ultra-low attachment surface flask, thereby producing neurospheres (cell masses containing neural stem cells in an undifferentiated state). The neurospheres thus produced were dispersed by pipetting, and used in the membrane permeability test described below.

The membrane permeability test was carried out under the following conditions.
(1) Culture Vessel: Poly-D-Lysine coated chamber slides (BioCoat product from Becton-Dickson Japan (BD)) were used.
(2) Procedure: A cell suspension containing mouse neural stem cells (mNSC) from the above neurosphere was adjusted with the above growth medium to a concentration of 5×10⁴ cells/mL. Next, the above artificial peptide (one of above Samples 1 to 5) that had been labeled beforehand with fluorescein isothiocyanate (FITC) was added to this cell suspension to a peptide concentration of 1 µM. Next, 1 mL of a cell suspension containing this FITC-labeled peptide (one of above Samples 1 to 5) was added to each well in the above culture vessel. From such addition, specimens cultured for 0.5 hour, specimens cultured for 1 hour, specimens cultured for 2 hours, and specimens cultured for 6 hours at 37°C and under 5% CO₂ (i.e., the cell culture in the well) were each sampled and fixed with methanol. These methanol-fixed specimens (cells) were then encapsulated using Prolong® Gold Antifade Reagent (Invitrogen). Next, using a confocal laser scanning microscope, the localization of FITC-labeled peptide was confirmed for each specimen (the above specimens that were encapsulated after methanol fixation).

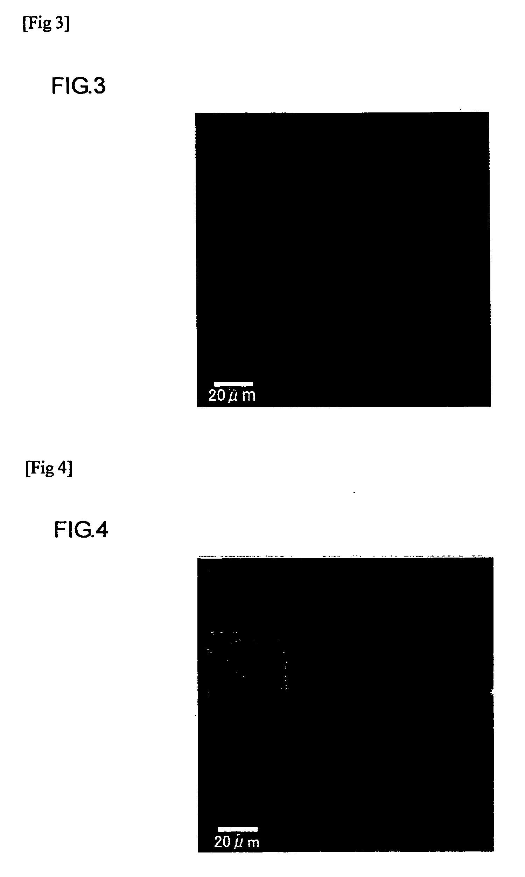

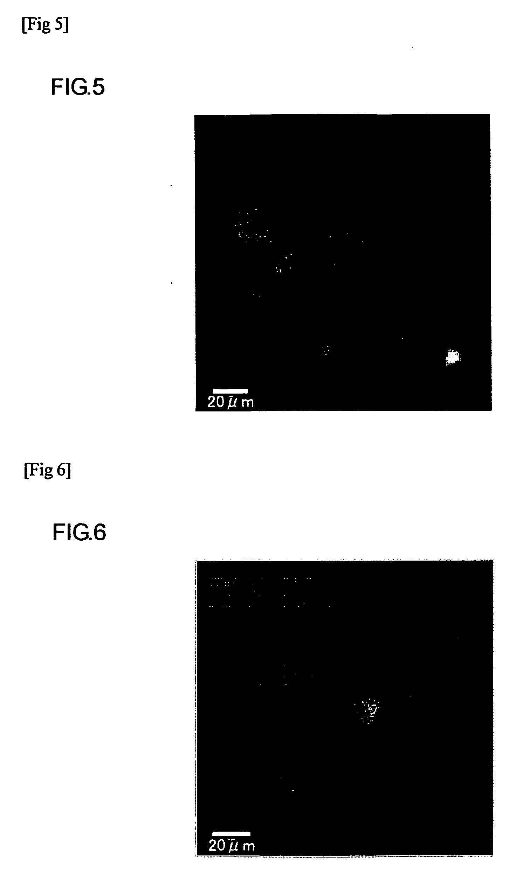

FIGS. 3 to 6 are micrographs showing the results for cell suspensions in which the FITC-labeled peptide of Sample 1 had been added. FIGS. 3, 4, 5 and 6 show the results for samples obtained after, respectively, 0.5 hour of culturing, 1 hour of culturing, 2 hours of culturing and 6 hours of culturing.

As is apparent from these micrographs, following addition, these peptides were confirmed to rapidly move into the cell from outside the cell by passing through the cell membrane, and furthermore to move into the nucleus (i.e., localize within the nucleus). That is, the total of five artificial peptides (Samples 1 to 5) having neuronal differentiation-inducing activities obtained in Example 1 were confirmed to exhibit excellent cell membrane permeabilities.

Moreover, although detailed data are not shown here, even in cases where the BC-box-related sequences (hSOCS-6, hASB-7, mASB-10, hASB-14) composed of a total of 15 amino acid residues included in the respective peptides of Samples 1 to 5 were changed to short sequences composed of only 10 amino acid residues from the N-terminus of these BC-box-related sequences, similar good neuronal differentiation-inducing activities were confirmed.

Several embodiments of the invention have been described in detail above, although these serve only to illustrate the invention and are not intended to limit the scope of the attached claims. Many variations and modifications of the above embodiments are encompassed by the art as recited in the appended claims.

## Claims

1. A method of transporting a peptide motif of interest into a nucleus of a eukaryotic cell from outside the cell, comprising:
chemically synthesizing a polypeptide chain having an amino acid sequence constituting the peptide motif of interest at an N-terminal end or a C-terminal end of a cell membrane-permeable nucleolar localization signal sequence defined by the amino acid sequence KKRTLRKNDRKKR (SEQ ID NO: 1);
adding the chemically synthesized polypeptide to a culture medium which includes the eukaryotic cell of interest or a tissue containing the cell;
culturing the eukaryotic cell or the tissue containing the cell in the medium containing the chemically synthesized polypeptide;
and
introducing the chemically synthesized polypeptide into the culturing eukaryotic cell by cell membrane permeability of the chemically synthesized polypeptide itself.

2. The method of claim 1, wherein a total number of amino acid residues constituting the synthesized polypeptide is not more than 500.

3. The method of claim 1 or 2, wherein the amino acid sequence constituting the peptide motif of interest is selected from any of SEQ ID NOS: 2 to 80.

4. The method of claim 3, wherein a total number of amino acid residues constituting the synthesized polypeptide is not more than 50.

5. The method of any one of claims 1 to 4, wherein the eukaryotic cell is a human stem cell or a non-human mammalian origin stem cell.

6. A chemically synthesized polypeptide having cell membrane permeability, and used for the purpose of transporting a peptide motif of interest into a nucleus of a eukaryotic cell from outside the cell, the polypeptide comprising a polypeptide chain synthesized so as to have, at an N-terminal end or a C-terminal end of a cell membrane-permeable nucleolar localization signal sequence defined by the amino acid sequence KKRTLRKNDRKKR (SEQ ID NO: 1), an amino acid sequence constituting the peptide motif of interest which does not, in nature, exist next to the signal sequence,
wherein the total number of amino acid residues constituting the chemically synthesized polypeptide is not more than 500.

7. The chemically synthesized polypeptide of claim 6, wherein the amino acid sequence constituting the peptide motif of interest is selected from any of SEQ ID NOS: 2 to 80.

8. The chemically synthesized polypeptide of claim 7, wherein a total number of amino acid residues constituting the synthesized polypeptide is not more than 50.

9. A method of producing a chemically synthesized polypeptide having cell membrane permeability, and used for transporting a peptide motif of interest into a nucleus of a eukaryotic cell from outside the cell, comprising:
selecting the peptide motif and an amino acid sequence constituting the motif;
designing a polypeptide chain having the amino acid sequence constituting the selected peptide motif at an N-terminal end or a C-terminal end of a cell membrane-permeable nucleolar localization signal sequence defined by the amino acid sequence KKRTLRKNDRKKR (SEQ ID NO: 1); wherein the polypeptide chain is designed in such a manner that a total number of amino acid residues is not more than 500; and
chemically synthesizing the designed polypeptide chain.

10. The production method of claim 9, wherein the amino acid sequence constituting the peptide motif of interest is selected from any of SEQ ID NOS: 2 to 80.

11. The production method of claim 10, wherein a total number of amino acid residues constituting the synthesized polypeptide is not more than 50.

## Patentansprüche

1. Verfahren zum Transportieren eines Peptidmotivs von Interesse in einen Zellkern einer eukaryotischen Zelle von außerhalb der Zelle, umfassend:
chemisches Synthetisieren einer Polypeptidkette, die eine Aminosäuresequenz aufweist, die das Peptidmotiv von Interesse an einem N-terminalen Ende oder einem C-terminalen Ende einer Zellmembran-permeablen nukleolären Lokalisierungssignalsequenz, die durch die Aminosäuresequenz KKRTLRKNDRKKR (SEQ ID NO: 1) definiert ist, bildet;
Hinzufügen des chemisch synthetisierten Polypeptids zu einem Kulturmedium, welches die eukaryotische Zelle von Interesse oder ein Gewebe, das die Zelle enthält, einschließt;
Kultivieren der eukaryotischen Zelle oder des Gewebes, das die Zelle enthält, in dem Medium, das das chemisch synthetisierte Polypeptid enthält; und
Einführen des chemisch synthetisierten Polypeptids in die kultivierende eukaryotische Zelle durch die Zellmembranpermeabilität des chemisch synthetisierten Polypeptids selbst.

2. Verfahren nach Anspruch 1, wobei eine Gesamtzahl von Aminosäureresten, die das synthetisierte Polypeptid bilden, nicht mehr als 500 beträgt.

3. Verfahren nach Anspruch 1 oder 2, wobei die Aminosäuresequenz, die das Peptidmotiv von Interesse bildet, aus einer der SEQ ID NOS: 2 bis 80 ausgewählt ist.

4. Verfahren nach Anspruch 3, wobei eine Gesamtzahl von Aminosäureresten, die das synthetisierte Polypeptid bilden, nicht mehr als 50 beträgt.

5. Verfahren nach einem der Ansprüche 1 bis 4, wobei die eukaryotische Zelle eine humane Stammzelle oder eine nicht-humane Stammzelle Säugetierursprungs ist.

6. Chemisch synthetisiertes Polypeptid, das eine Zellmembranpermeabilität aufweist und für den Zweck des Transportierens eines Peptidmotivs von Interesse in einen Zellkern einer eukaryotischen Zelle von außerhalb der Zelle verwendet wird, das Polypeptid umfassend eine Polypeptidkette, die so synthetisiert ist, dass sie an einem N-terminalen Ende oder einem C-terminalen Ende einer Zellmembran-permeablen nukleolären Lokalisierungssignalsequenz, die durch die Aminosäuresequenz KKRTLRKNDRKKR (SEQ ID NO: 1) definiert ist, eine Aminosäuresequenz aufweist, die das Peptidmotiv von Interesse bildet, welches in der Natur nicht neben der Signalsequenz vorkommt,
wobei die Gesamtzahl von Aminosäureresten, die das chemisch synthetisierte Polypeptid bilden, nicht mehr als 500 beträgt.

7. Chemisch synthetisiertes Polypeptid nach Anspruch 6, wobei die Aminosäuresequenz, die das Peptidmotiv von Interesse bildet, aus einer der SEQ ID NOS: 2 bis 80 ausgewählt ist.

8. Chemisch synthetisiertes Polypeptid nach Anspruch 7, wobei eine Gesamtzahl von Aminosäureresten, die das synthetisierte Polypeptid bilden, nicht mehr als 50 beträgt.

9. Verfahren zum Herstellen eines chemisch synthetisierten Polypeptids, das eine Zellmembranpermeabilität aufweist und für den Zweck des Transportierens eines Peptidmotivs von Interesse in einen Zellkern einer eukaryotischen Zelle von außerhalb der Zelle verwendet wird, umfassend:
Auswählen des Peptidmotivs und einer Aminosäuresequenz, die das Motiv bildet; Entwerfen einer Polypeptidkette, die eine Aminosäuresequenz aufweist, die das ausgewählte Peptidmotiv an einem N-terminalen Ende oder einem C-terminalen Ende einer Zellmembran-permeablen nukleolären Lokalisierungssignalsequenz, die durch die Aminosäuresequenz KKRTLRKNDRKKR (SEQ ID NO: 1) definiert ist, bildet;
wobei die Polypeptidkette in einer solchen Weise entworfen wird, dass eine Gesamtzahl von Aminosäureresten nicht mehr als 500 beträgt; und
chemisches Synthetisieren der entworfenen Polypeptidkette.

10. Herstellungsverfahren nach Anspruch 9, wobei die Aminosäuresequenz, die das Peptidmotiv von Interesse bildet, aus einer der SEQ ID NOS: 2 bis 80 ausgewählt ist.

11. Herstellungsverfahren nach Anspruch 10, wobei eine Gesamtzahl von Aminosäureresten, die das synthetisierte Polypeptid bilden, nicht mehr als 50 beträgt.

## Revendications

1. Procédé de transport d'un motif peptidique d'intérêt dans le noyau d'une cellule eucaryote à partir de l'extérieur de la cellule, comprenant:
la synthèse chimique d'une chaîne polypeptidique ayant une séquence d'acides aminés constituant le motif peptidique d'intérêt à l'extrémité N-terminale ou à l'extrémité C-terminale d'une séquence signal à localisation nucléolaire perméable à la membrane cellulaire, définie par la séquence d'acides aminés KKRTLRKNDRKKR (SEQ ID NO: 1);
l'addition du polypeptide synthétisé par voie chimique à un milieu de culture qui comprend la cellule eucaryote d'intérêt ou un tissu contenant la cellule;
la culture de la cellule eucaryote ou du tissu contenant la cellule dans le milieu contenant le polypeptide synthétisé par voie chimique; et
l'introduction du polypeptide synthétisé par voie chimique dans la cellule eucaryote en culture par perméabilité de la membrane cellulaire au polypeptide synthétisé par voie chimique lui-même.

2. Procédé selon la revendication 1, dans lequel le nombre total de résidus d'acides aminés constituant le polypeptide synthétisé n'est pas supérieur à 500.

3. Procédé selon la revendication 1 ou 2, dans lequel la séquence d'acides aminés constituant le motif peptidique d'intérêt est choisie parmi toutes les SEQ ID NO: 2 à 80.

4. Procédé selon la revendication 3, dans lequel le nombre total de résidus d'acides aminés constituant le polypeptide synthétisé n'est pas supérieur à 50.

5. Procédé selon l'une quelconque des revendications 1 à 4, dans lequel la cellule eucaryote est une cellule de souche humaine ou une cellule de souche d'origine mammalienne non humaine.

6. Polypeptide synthétisé par voie chimique ayant une perméabilité à la membrane cellulaire, et utilisé dans le but de transporter un motif peptidique d'intérêt dans le noyau d'une cellule eucaryote à partir de l'extérieur de la cellule, le polypeptide comprenant une chaîne polypeptidique synthétisée de manière à avoir, à l'extrémité N-terminale ou à l'extrémité C-terminale d'une séquence signal à localisation nucléolaire perméable à la membrane cellulaire définie par la séquence d'acides aminés KKRTLRKNDRKKR (SEQ ID NO: 1), une séquence d'acides aminés constituant le motif peptidique d'intérêt, qui n'existe pas dans la nature, voisine de la séquence signal,
le nombre total de résidus d'acides aminés constituant le polypeptide synthétisé par voie chimique n'étant pas supérieur à 500.

7. Polypeptide synthétisé par voie chimique selon la revendication 6, dans lequel la séquence d'acides aminés constituant le motif peptidique d'intérêt est choisi parmi toutes les SEQ ID NO: 2 à 80.

8. Polypeptide synthétisé par voie chimique selon la revendication 7, dans lequel le nombre total de résidus d'acides aminés constituant le polypeptide synthétisé n'est pas supérieur à 50.

9. Procédé de production d'un polypeptide synthétisé par voie chimique ayant une perméabilité à la membrane cellulaire, et utilisé dans le but de transporter un motif peptidique d'intérêt dans le noyau d'une cellule eucaryote à partir de l'extérieur de la cellule, comprenant:
le choix du motif peptidique et d'une séquence d'acides aminés constituant le motif;
la conception d'une chaîne polypeptidique ayant la séquence d'acides aminés constituant le motif peptidique choisi à l'extrémité N-terminale ou à l'extrémité C-terminale d'une séquence signal à localisation nucléolaire perméable à la membrane cellulaire définie par la séquence d'acides aminés KKRTLRKNDRKKR (SEQ ID NO: 1), la chaîne polypeptidique étant conçue de manière que le nombre total de résidus d'acides aminés ne soit pas supérieur à 500; et
la synthèse chimique de la chaîne polypeptidique conçue.

10. Procédé de production selon la revendication 9, dans lequel la séquence d'acides aminés constituant le motif peptidique d'intérêt est choisi parmi toutes les SEQ ID NO: 2 à 80.

11. Procédé de production selon la revendication 10, dans lequel le nombre total de résidus d'acides aminés constituant le polypeptide synthétisé n'est pas supérieur à 50.
